(19) Europäisches Patentamt
European Patent Office
Office européen des brevets

(11) Veröffentlichungsnummer: **0 031 877**
**B1**

(12) # EUROPÄISCHE PATENTSCHRIFT

(45) Veröffentlichungstag der Patentschrift: **02.02.83**

(21) Anmeldenummer: **80106352.0**

(22) Anmeldetag: **18.10.80**

(51) Int. Cl.³: **C 07 F 9/50**, C 07 F 15/00, B 01 J 31/00, C 07 C 102/00, C 07 C 103/46

(54) **Optisch aktive 1,2-Bis(diphenylphosphino)-verbindungen, diese als chirale Liganden enthaltende Metallkomplexe und deren Verwendung.**

(30) Priorität: **08.01.80 DE 3000445**

(43) Veröffentlichungstag der Anmeldung: **15.07.81 Patentblatt 81/28**

(45) Bekanntmachung des Hinweises auf die Patenterteilung: **02.02.83 Patentblatt 83/5**

(84) Benannte Vertragsstaaten: **BE CH FR GB IT LI NL**

(73) Patentinhaber: **Degussa Aktiengesellschaft, Weissfrauenstrasse 9, D-6000 Frankfurt am Main 1 (DE)**

(72) Erfinder: **Bergstein, Wolfgang, Dr. Dipl.-Chem., Lindenstrasse 10, D-6458 Rodenbach 1 (DE)**
Erfinder: **Kleemann, Axel Dr. Dipl.-Chem, Greifenhagenstrasse 9, D-6450 Hanau 9 (DE)**
Erfinder: **Martens, Jürgen Dr. Dipl.-Chem, Hochstrasse 5, D-8755 Alzenau (DE)**

(56) Entgegenhaltungen:
DE-A-2 161 200
GB-A-2 014 138

„Journal of the American Chemical Society", Bd. 100, Nr. 17, 16. August 1978, S. 5491-5494, M. D. Fryzuk *et al.*: „Asymmetric Synthesis. An Asymmetric Homogeneous Hydrogenation Catalyst Which Breeds Its Own Chirality".
„Journal of the Organic Chemistry", Bd. 44, Nr. 10, 1979, S. 1729-1731, R. B. King *et al.*: „1,2-Bis(diphenylphosphino)-1-phenylethane: A Chiral Ditertiary Phosphine Derived from Mandelic Acid Used as a Ligand in Asymmetric Homogeneous Hydrogenation Catalysts".
„Journal of the American Chemical Society", Bd. 99, Nr. 11, 14. September 1977, S. 6262-7, M. D. Fryzuk *et al.*: „Asymmetric Synthesis. Production of Optically Active Amino Acids by Caralytic Hydrogenation".

EP 0 031 877 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Optisch aktive 1,2-Bis(diphenylphosphino)verbindungen, diese als chirale Liganden enthaltende Metallkomplexe und deren Verwendung

Die Erfindung betrifft neue optisch aktive 1,2-Bis(diphenylphosphino)verbindungen, Rhodiumkomplexe, die diese als chirale Liganden enthalten, und die Verwendung dieser Rhodiumkomplexe als Katalysatoren für die homogene asymmetrische Hydrierung unsubstituierter oder β-substituierter α-Acylamidoacrylsäure bzw. deren Derivate.

Aus „J. Am. Chem. Soc.", *100* (1978), S. 5491 bis 5494, sind bereits optisch aktives 1,2-Bis(diphenylphosphino)propan, ein dieses als chiralen Liganden enthaltender Rhodiumkomplex und dessen Verwendung als Katalysator für die homogene asymmetrische Hydrierung unsubstituierter oder β-substituierter α-Acylamidoacrylsäure und deren Derivate bekannt. Aus „J. Org. Chem.", *44* (1979), S. 1729 bis 1731, sind ferner bereits optisch aktives 1,2-Bis(diphenylphosphino)-1-phenyläthan, ein dieses als chiralen Liganden enthaltender Rhodiumkomplex und dessen Verwendung als Katalysator für die homogene asymmetrische Hydrierung von α-Acylamidozimtsäure und deren Derivaten bekannt. Mit den bekannten Rhodiumkomplexen werden jedoch bei der asymmetrischen Hydrierung nicht vollbefriedigende optische Ausbeuten erzielt.

Durch die vorliegende Erfindung soll daher die Aufgabe gelöst werden, die Enantioselektivität bei der homogenen asymmetrischen Hydrierung unsubstituierter oder β-substituierter α-Acylamidoacrylsäure bzw. deren Derivate in Gegenwart von chirale Liganden enthaltenden Rhodiumkomplexen zu verbessern.

Gegenstand der Erfindung sind daher neue optisch aktive 1,2-Bis(diphenylphosphino)verbindungen der allgemeinen Formel

```
R-CH——CH2
  |    |          (I)
P(Ph)2 P(Ph)2
```

in der Ph einen Phenylrest, und R einen Isopropyl-, einen Isobutyl-, einen sekundären Butyl- oder einen Benzylrest bedeuten.

Die erfindungsgemässen Verbindungen der Formel I können auf folgende Weise hergestellt werden:

Eine optisch aktive α-Hydroxycarbonsäure der allgemeinen Formel

```
R-CH-COOH
  |             (V)
  OH
```

in der R die angegebene Bedeutung hat, wird als freie Säure oder als Ester in einem Äther, beispielsweise Tetrahydrofuran, unter Kühlung mit Lithium/Aluminium/Hydrid zu dem enantiomerenreinen 1,2-Diol der allgemeinen Formel

```
R-CH-CH2
  |  |          (VI)
  OH OH
```

reduziert, in der R wieder die angegebene Bedeutung hat. Dabei tritt keine Racemisierung ein. Durch Umsetzung dieses 1,2-Diols mit mindestens der zweifachen molaren Menge an p-Toluolsulfonsäurechlorid unter Kühlung in Pyridin als Lösungsmittel erhält man das entsprechende 1,2-Ditosylat. Auch bei dieser Umsetzung tritt keine Racemisierung ein. Das gebildete 1,2-Ditosylat wird schliesslich in einem Äther, beispielsweise Tetrahydrofuran, bei Temperaturen zwischen −20 und +25°C mit mindestens der stöchiometrischen Menge eines Alkalimetalldiphenylphosphids in die entsprechende optisch aktive 1,2-Bis(diphenylphosphino)verbindung umgewandelt. Die so erhältlichen optisch aktiven Verbindungen der Formel I können als chirale Liganden in Metallkomplexen dienen, die als Zentralatom Rhodium enthalten.

Ein weiterer Gegenstand der Erfindung sind daher Metallkomplexe, die Rhodium als Zentralatom und eine erfindungsgemässe Verbindung der Formel I als chiralen Liganden enthalten.

Derartige Rhodiumkomplexe können *in situ* hergestellt werden durch Umsetzung einer Verbindung der Formel I mit einem Komplex der allgemeinen Formel

$$[Rh(en)_2X]_2 \qquad (II)$$

in der $(en)_2$ zwei Moleküle eines Monoolefins, wie Äthylen oder Cycloocten, oder ein Molekül eines Diolefins, wie Butadien, Cyclooctadien oder Norbornadien, und X Chlor, Brom oder Jod bedeuten, im molaren Verhältnis 2:1, oder mit einem Komplex der allgemeinen Formel

$$[Rh(en)_2Y] \qquad (III)$$

in der $(en)_2$ die angegebene Bedeutung hat, und Y einen Acetonylacetonat- oder einen Carboxylatrest, beispielsweise einen Acetat-, Propionat- oder Benzoatrest, bedeutet, im molaren Verhältnis 1:1. Die Umsetzung erfolgt in diesem Falle zweckmässigerweise direkt in dem Lösungsmittel, in dem später eine Hydrierung in Gegenwart des gebildeten Rhodiumkomplexes vorgenommen werden soll, beispielsweise in Methanol oder Äthanol.

Kationische Rhodiumkomplexe der allgemeinen Formel

$$[Rh(en)_2A]^+ Z^- \qquad (IV)$$

in der $(en)_2$ wieder die bereits angegebene Bedeutung hat, A eine erfindungsgemässe optisch aktive Verbindung der Formel I, und $Z^-$ ein Tetrafluoroborat-, Hexafluorophosphat- oder Perchloratanion bedeuten, können hergestellt werden durch die vorstehend beschriebene Umsetzung einer Verbindung der Formel I mit einem Rhodiumkomplex der Formel II oder III und durch anschliessende Zugabe eines Alkalimetall- oder Silbersalzes der Tetrafluoroborsäure, Hexafluorophosphorsäure oder Perchlorsäure, oder von freier Perchlorsäure. Die Umsetzung wird zweckmässigerweise in einem Alkohol, wie Methanol oder Äthanol, vorgenommen. Das Salz bzw. die freie Perchlorsäure wird zweckmässigerweise in Form einer möglichst konzentrierten wässerigen Lösung zugegeben. Dabei ist es nicht unbedingt erforderlich, die Verbindung der Formel I in reiner Form einzusetzen. Man kann sie vielmehr auch in der unmittelbar bei der Herstellung anfallenden verunreinigten Form, häufig als Öl, einsetzen und die Reinigung mit der Herstellung des kationischen Rhodiumkomplexes in einem Arbeitsgang verbinden. Das kann in der Weise geschehen, dass man den Rhodiumkomplex der Formel II oder III mit der äquimolaren (bezogen auf Rhodium) Menge der unreinen Verbindung der Formel I in einem niederen Alkohol als Lösungsmittel umsetzt, das Salz oder die freie Perchlorsäure zugibt und anschliessend durch Zusatz von Wasser die Verunreinigungen ausfällt. Dabei wird solange Wasser zugesetzt, bis die über der sich bildenden Ausfällung stehende Komplexlösung die für die kationischen Rhodiumkomplexe charakteristische orange-rote Färbung aufweist. Dann wird diese Komplexlösung von dem gebildeten Niederschlag abgetrennt und der Komplex entweder durch Einengen der Lösung oder durch weitere Zugabe von Wasser in fester Form gewonnen.

Ein weiterer Gegenstand der Erfindung ist die Verwendung der beschriebenen Rhodiumkomplexe, die eine Verbindung der Formel I als chiralen Liganden enthalten, als Katalysatoren für die homogene asymmetrische Hydrierung unsubstituierter oder β-substituierter α-Acylamidoacrylsäure bzw. deren Derivate.

Als Substrate für die asymmetrische Hydrierung können beispielsweise α-Acylamidoacrylsäuren und deren Derivate der allgemeinen Formel

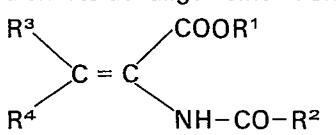

(VII)

dienen. In dieser Formel bedeuten $R^1$ Wasserstoff, ein Alkalimetall oder einen niederen Alkylrest mit 1 bis 4, vorzugsweise 1 bis 2, Kohlenstoffatomen und $R^2$ einen niederen Alkylrest mit 1 bis 4, vorzugsweise 1 bis 2, Kohlenstoffatomen oder einen Phenylrest. $R^3$ und $R^4$ können gleich oder verschieden sein und Wasserstoff, einen geradkettigen oder verzweigten Alkylrest mit 1 bis 10 Kohlenstoffatomen, einen unsubstituierten oder in 3- und/oder 4-Stellung durch Hydroxyl-, Alkoxy- oder Acyloxygruppen substituierten Phenylrest oder einen unsubstituierten oder in 6-Stellung durch eine Methylgruppe oder durch Chlor substituierten Indolylrest bedeuten.

Durch die asymmetrische Hydrierung werden die genannten prochiralen Substrate in die entsprechenden optisch aktiven α-Acylamidocarbonsäuren umgewandelt, die sich in an sich bekannter Weise zu den entsprechenden α-Aminocarbonsäuren verseifen lassen. Wurde die im verwendeten Rhodiumkomplex enthaltene erfindungsgemässe optisch aktive 1,2-Bis(diphenylphosphino)verbindung ursprünglich aus einer L-α-Hydroxycarbonsäure hergestellt, so entsteht in grossem Überschuss das L-Enantiomere der optisch aktiven α-Acylamidocarbonsäure; wurde dagegen von der entsprechenden D-α-Hydroxycarbonsäure ausgegangen, so entsteht in grossem Überschuss das D-Enantiomere. In beiden Fällen können die gewünschten Enantiomeren optisch nahezu vollständig rein erhalten werden.

Die Hydrierung erfolgt in den für Hydrierungen üblichen Lösungsmitteln, wie Alkoholen und Äthern oder deren Mischungen mit aliphatischen oder aromatischen Kohlenwasserstoffen oder mit Wasser. Die Substratkonzentration kann von einer 0,001-molaren bis zu einer an Substrat übersättigten Lösung reichen. Der Wasserstoffdruck kann zwischen Normaldruck und 50 bar, vorzugsweise zwischen Normaldruck und 25 bar, liegen, die Reaktionstemperatur zwischen −20 und +50° C. Vorzugsweise wird die Hydrierung bei Raumtemperatur vorgenommen. Die eine Verbindung der Formel I als chiralen Liganden enthaltenden Rhodiumkomplexe werden zweckmässig in solcher Menge eingesetzt, dass das Molverhältnis von Substrat zu Katalysator im Bereich zwischen 1:1 und 50 000:1, vorzugsweise zwischen 500:1 bis 10 000:1, liegt.

Wegen der Empfindlichkeit der erfindungsgemässen optisch aktiven 1,2-Bis(diphenylphosphino)verbindungen und der sie als chirale Liganden enthaltenden Metallkomplexe gegenüber Sauerstoff ist es zweckmässig, alle Reaktionen in Schutzgasatmosphäre, z.B. unter Stickstoff oder Argon, durchzuführen und auch die jeweiligen Reaktionsprodukte unter Schutzgas aufzubewahren. Ausserdem ist es empfehlenswert, auch die Hydrierungen unter anaeroben Bedingungen vorzunehmen.

Bei der Hydrierung der prochiralen Substrate der Formel VII werden in allen Fällen optisch aktive N-Acyl-α-aminocarbonsäuren erhalten, die sich leicht über die entsprechenden optisch aktiven α-Aminocarbonsäuren in die entsprechenden optisch aktiven α-Hydroxycarbonsäuren umwandeln lassen. Sofern in den zu hydrierenden Substraten die Voraussetzungen zur Bildung eines der Formel I entsprechenden Kohlenstoffgerüstes gegeben sind, kann daher ein Teil des Hydrierungsproduktes für die Herstellung neuer Mengen an Verbindungen der Formel I und damit neuer Mengen an erfindungsgemässen Metallkomplexen abgezweigt werden.

Die Erfindung wird durch die nachfolgenden Beispiele näher erläutert, ohne dass dadurch der Umfang der Erfindung beschränkt werden soll.

*Beispiel 1:*

*Darstellung von (R)-1,2-Bis(diphenylphosphino)-3-phenylpropan*

*a) (S)-1,2-Dihydroxy-3-phenylpropan*

Zu einer auf 0° C gekühlten Suspension von 50 g $LiAlH_4$ in 750 ml Tetrahydrofuran (THF) wurden innerhalb von 3 h 83,1 g (S)-Phenylmilchsäure, gelöst in 250 ml THF, zugetropft. Die Reaktionsmischung wurde 2 h unter Rückfluss gekocht,

nach Abkühlen portionsweise mit Wasser und 43 ml 4N-NaOH versetzt und 0,5 h unter Rückfluss gekocht. Der weisse Niederschlag wurde abfiltriert und sorgfältig mit heissem THF ausgewaschen. Von den vereinigten Filtraten wurde das Lösungsmittel abdestilliert und das Rohprodukt fraktionierend destilliert. Man erhält 64,6 g (S)-1,2-Dihydroxy-3-phenylpropan vom Siedepunkt 101-102°C (0,001 mbar).

$$[\alpha]_D^{20} = -36° \text{ (c = 1/EtOH)}$$

*Elementaranalyse:*

| | | |
|---|---|---|
| gefunden: | C 71,01 | H 7,91% |
| berechnet: | C 71,03 | H 7,95% |

Ausbeute: 85% der Theorie.

*b) (S)-3-Phenylpropandiol-1,2-ditosylat*

31,8 g des unter a) hergestellten Diols wurden in 20 ml Pyridin gelöst und in 0,75 h zu einer auf 0°C gekühlten Mischung aus 83,9 g p-Toluolsulfonsäurechlorid und 105 ml Pyridin getropft. Der Ansatz wurde über Nacht bei Raumtemperatur nachgerührt und anschliessend mit 80 ml Eiswasser versetzt. Danach wurde die Mischung auf 400 ml Eis gegeben, das mit 100 ml konzentrierter Salzsäure versetzt war, und das pastöse Rohprodukt mit Methylenchlorid abgetrennt. Der Methylenchloridextrakt wurde zunächst mit verdünnter Salzsäure, dann mit Wasser bis zum Neutralpunkt gewaschen. Das Lösungsmittel wurde abdestilliert. Es verblieben 91 g kristallines (S)-3-Phenylpropandiol-1,2-ditosylat.

Ausbeute: 98,8% der Theorie.

$$[\alpha]_D^{20} = -26,6° \text{ (c = 1/CHCl}_3\text{)}$$

*Elementaranalyse:*

| | | | |
|---|---|---|---|
| gefunden: | C 59,75 | H 5,38 | S 14,04% |
| berechnet: | C 59,98 | H 5,25 | S 13,92% |

*c) (R)-1,2-Bis(diphenylphosphino)-3-phenylpropan*

Zu 37,8 g Natriumdiphenylphosphid, gelöst in 450 ml THF, wurde bei einer Temperatur zwischen −10 und −15°C eine THF-Lösung von 25,7 g des unter b) hergestellten Ditosylats getropft. Der ausgefallene Niederschlag wurde abfiltriert und gründlich mit THF gewaschen.

Die Hälfte des Filtrats wurde 1 h mit 200 ml Wasser gerührt. Nach Abdestillieren des THF im Vakuum wurde das angefallene Öl mit Äther extrahiert. Die vereinigten Ätherextrakte wurden mit $Na_2SO_4$ getrocknet. Nach Abdestillieren des Äthers und Trocknen des Rückstands im Vakuum verblieben 12,3 g eines Öls, das einen Drehwert von

$[\alpha]_D^{20} = +45,91°$ (c = 1,3634/$CHCl_3$) besass.

*Elementaranalyse:*

| | | | |
|---|---|---|---|
| gefunden: | C 81,08 | H 6,26 | P 12,56% |
| berechnet: | C 81,13 | H 6,19 | P 12,68% |

$^{31}$P-NMR 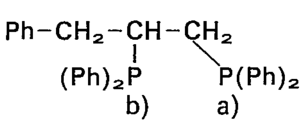

Chemische Verschiebung:
a) $\delta = -4,9$ ppm
b) $\delta = -22,6$ ppm
Kopplungskonstante: $J_{P\text{-}P} = 31,1$ Hz
Laut $^{31}$P-NMR-Spektrum war das Produkt 67%ig.

*Beispiel 2:*

*Darstellung von (S)-1,2-Bis(diphenylphosphino)-3-phenylpropan*

*a) (R)-1,2-Dihydroxy-3-phenylpropan*

Das Beispiel 1 a) wurde wiederholt mit dem Unterschied, dass anstelle der (S)-Phenylmilchsäure (R)-Phenylmilchsäure eingesetzt wurde.

Ausbeute an (R)-1,2-Dihydroxy-3-phenylpropan: 84,5% der Theorie.

*Elementaranalyse:*

| | | |
|---|---|---|
| gefunden: | C 70,97 | H 7,94% |
| berechnet: | C 71,03 | H 7,95% |

$$[\alpha]_D^{20} = +35,8° \text{ (c = 1/EtOH)}$$

*b) (R)-3-Phenyl-1,2-propandiolditosylat*

Das unter a) hergestellte Diol wurde, wie im Beispiel 1 b) angegeben, umgesetzt. Dabei wurde das (R)-3-Phenyl-1,2-propandiolditosylat erhalten.

Ausbeute: 96,8% der Theorie.

*Elementaranalyse:*

| | | | |
|---|---|---|---|
| gefunden: | C 59,84 | H 5,19 | S 14,08% |
| berechnet: | C 59,98 | H 5,25 | S 13,92% |

$$[\alpha]_D^{20} = +26,3° \text{ (c = 1/CHCl}_3\text{)}$$

*c) (S)-1,2-Bis(diphenylphosphino)-3-phenylpropan*

Das unter b) erhaltene (R)-3-Phenyl-1,2-propandiolditosylat wurde, wie im Beispiel 1 c) beschrieben, zum (S)-Bis-1,2-(diphenylphosphino)-3-phenylpropan umgesetzt.

*Elementaranalyse:*

| | | | |
|---|---|---|---|
| gefunden: | C 81,28 | H 6,27 | P 12,81% |
| berechnet: | C 81,13 | H 6,19 | P 12,68% |

$$[\alpha]_D^{20} = -44,2° \text{ (c = 1,2074/CHCl}_3\text{)}$$

*Beispiel 3:*

*Darstellung von (R)-1,2-Bis(diphenylphosphino)-3-methylbutan*

*a) (S)-1,2-Dihydroxy-3-methylbutan*

57,8 g (S)-2-Hydroxyisovaleriansäure, gelöst in 250 ml THF, wurden in 3 h zu einer auf 0°C gekühlten Suspension von 46,4 g Lithium/Aluminium/Hydrid in 700 ml THF getropft. Es wurde 1 h unter Rückfluss gekocht, abgekühlt, und das überschüssige $LiAlH_4$ durch portionsweise Zugabe von $H_2O$ und verdünnter NaOH zerstört. Die Suspension wurde noch einmal kurz aufgekocht und der Niederschlag abfiltriert. Dieser wurde mit heissem THF ausgewaschen. Die vereinigten Filtrate wurden am Rotationsverdampfer vom Lösungsmittel befreit und der Rückstand im Ölpumpenvakuum destilliert. Man erhält 43,0 g (S)-1,2-Dihydroxy-3-methylbutan vom Siedepunkt 62°C

(0,5 mbar), entsprechend einer Ausbeute von 84,5% der Theorie.

*Elementaranalyse:*

| | | | |
|---|---|---|---|
| gefunden: | C 57,76 | H 11,80% | |
| berechnet: | C 57,66 | H 11,61% | |

$[\alpha]_D^{20} = +9{,}9° \ (c = 1/CHCl_3)$

Das NMR-Spektrum entspricht dem Produkt.

*b) (S)-3-Methyl-1,2-butandiolditosylat*

165 g frisch umkristallisiertes p-Toluolsulfonsäurechlorid wurden in 200 ml Pyridin suspendiert, auf 0°C gekühlt und während 1 h mit einer Lösung von 39,8 g des unter a) hergestellten (S)-Diols, gelöst in 20 ml Pyridin, versetzt. Der Ansatz wurde über Nacht nachgerührt. Anschliessend wurden 160 ml Eiswasser zugegeben und auf 200 ml konzentrierte Salzsäure in 800 ml Eiswasser gegossen. Das Produkt wurde mit insgesamt 600 ml Methylenchlorid extrahiert. Der Extrakt wurde mit 0,5 N-Salzsäure und nachfolgend mit $H_2O$ bis zum Neutralpunkt gewaschen, mit $Na_2SO_4$ getrocknet und am Rotationsverdampfer vom Lösungsmittel befreit. Es verblieben 149 g, entsprechend einer Ausbeute von 94,3% der Theorie, eines öligen, aber schnell kristallisierenden Rückstandes. Der Drehwert des so erhaltenen (S)-3-Methyl-1,2-butandiolditosylats beträgt

$[\alpha]_D^{20} = -19{,}2° \ (c = 4/EtOH)$

*Elementaranalyse:*

| | | | |
|---|---|---|---|
| gefunden: | C 55,31 | H 5,89 | S 15,32% |
| berechnet: | C 55,32 | H 5,86 | S 15,54% |

Das NMR-Spektrum entspricht dem Produkt.

*c) (R)-1,2-Bis(diphenylphosphino)-3-methylbutan*

Unter Argon wurden 70,5 g Natriumdiphenylphosphid in 400 ml THF gelöst, auf −20°C gekühlt und innerhalb von 2 h mit einer Lösung von 45,4 g des unter b) hergestellten (S)-Ditosylats in 150 ml THF versetzt. Die Lösung wurde 3 h bei −5°C und dann über Nacht bei Raumtemperatur nachgerührt. Es wurden 300 ml sauerstofffreies Wasser zugegeben, das THF unter vermindertem Druck abdestilliert, und die wässerige Phase mit Äther extrahiert. Nach Abdestillieren des Äthers wurde der Rückstand in warmem Äthanol aufgenommen, abgekühlt und mehrmals mit Äthanol behandelt, bis sich das Diphosphan in Form farbloser Kristalle abschied. Das kristalline (R)-1,2-Bis(diphenylphosphino)-3-methylbutan hat einen Schmelzpunkt von 76-77°C und einen Drehwert

$[\alpha]_D^{20} = +98{,}1° \ (c = 0{,}626/CHCl_3)$

*Elementaranalyse:*

| | | | |
|---|---|---|---|
| gefunden: | C 78,89 | H 7,00 | P 14,12% |
| berechnet: | C 79,07 | H 6,87 | P 14,06% |

Das NMR-Spektrum entspricht dem Produkt.

*Beispiel 4:*

*Darstellung von (S)-1,2-Bis(diphenylphosphino)-3-methylbutan*

*a) (R)-1,2-Dihydroxy-3-methylbutan*

wird ausgehend von (R)-2-Hydroxyisovaleriansäure analog zum S-Isomeren [s. Beispiel 3 a)] hergestellt.

Die Ausbeute beträgt 86,5% der Theorie.

*Elementaranalyse:*

| | | | |
|---|---|---|---|
| gefunden: | C 57,80 | H 11,82% | |
| berechnet: | C 57,66 | H 11,61% | |

$[\alpha]_D^{20} = -10{,}0° \ (c = 1/CHCl_3)$

*b) (R)-3-Methyl-1,2-butandiolditosylat*

wird aus (R)-1,2-Dihydroxy-3-methylbutan analog zum S-Isomeren [s. Beispiel 3 b)] hergestellt.

Die Ausbeute beträgt 87,8% der Theorie.

*Elementaranalyse:*

| | | | |
|---|---|---|---|
| gefunden: | C 55,48 | H 5,90 | S 15,56% |
| berechnet: | C 55,32 | H 5,86 | S 15,54% |

$[\alpha]_D^{20} = +19{,}1° \ (c = 4/EtOH)$

*c) (S)-1,2-Bis(diphenylphosphino)-3-methylbutan*

wird aus (R)-3-Methyl-1,2-butandiolditosylat analog zum (R)-Isomeren [s. Beispiel 3 c)] hergestellt.

*Elementaranalyse:*

| | | | |
|---|---|---|---|
| gefunden: | C 78,92 | H 6,72 | P 14,16% |
| berechnet: | C 79,07 | H 6,87 | P 14,06% |

$[\alpha]_D^{20} = -98{,}0° \ (c = 0{,}630/CHCl_3)$

*Beispiel 5:*

*Darstellung von Cyclooctadien-[(R)-1,2-bis-(diphenylphosphino)-3-phenylpropan]rhodium-tetrafluoroborat*

1,52 g des nach Beispiel 1 c) hergestellten (R)-1,2-Bis(diphenylphosphino)-3-phenylpropans, das entspricht 2,08 mmol 100%igem Produkt, wurden in 100 ml Methanol gelöst, mit 0,75 g festem $[Rh(COD)Cl]_2$ versetzt und 2,5 h gerührt. 0,75 g $NaBF_4$, gelöst in 5 ml $H_2O$, wurden langsam zugetropft. Nach 2 h wurde solange Wasser zugegeben, bis die Ausfällung eines schmutzig gelben Niederschlages beendet war und die überstehende Lösung eine orange-rote Farbe angenommen hatte. Der Niederschlag wurde abgetrennt und zum Filtrat solange weiter Wasser zugegeben, bis kein Niederschlag mehr ausfiel. Der orangefarbene Niederschlag wurde abgefrittet, mit Wasser gewaschen und getrocknet. Man erhält 1,2 g des Komplexes, entsprechend einer Ausbeute von 73% der Theorie.

*Elementaranalyse:*

| | | | |
|---|---|---|---|
| gefunden: | B 1,28 | C 62,36 | H 5,79% |
| berechnet: | B 1,37 | C 62,62 | H 5,38% |
| gefunden: | F 9,54 | Rh 13,01 | P 7,95% |
| berechnet: | F 9,66 | Rh 13,08 | P 7,88% |

$^{31}P$-NMR 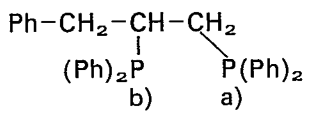

Chemische Verschiebung:

a) $\delta = +42,5$ ppm

b) $\delta = +55,9$ ppm

Kopplungskonstanten:

$J_{P-Rh}$: 147,1 Hz

$J_{P-Rh}$: 150,1 Hz

$J_{P-P}$: 31,7 Hz

*Beispiel 6:*

Zu 54,6 ml einer gesättigten Lösung von α-Acetamidozimtsäure in zwei Teilen Äthanol und einem Teil Benzol (0,89 g/10 ml ≙ 23,6 mmol) wurden 10,3 mg des im Beispiel 5 hergestellten Komplexes gegeben und in einen Autoklaven gesaugt. Es wurden 20 bar Wasserstoffdruck aufgepresst. Nach 1,5 h Schütteln bei Raumtemperatur war die Reaktion beendet.

Zur Entfernung des Katalysators wurde das Reaktionsgemisch 15 min mit 1,5 g eines sauren Ionenaustauscherharzes gerührt. Nach Abfiltrieren des Ionenaustauscherharzes und Abdestillieren des Lösungsmittels unter vermindertem Druck konnten 4,8 g N-Acetyl-(S)-phenylalanin mit dem Drehwert

$$[\alpha]_D^{20} = +46,8° \ (c = 1/95\% \ EtOH)$$

isoliert werden.

Bezogen auf den aus der Literatur bekannten Drehwert

$$[\alpha]_D^{20} = +47,5° \ (c = 1/95\% \ EtOH)$$

entspricht das einer optischen Ausbeute von 99% bei einem Substrat/Katalysator-Verhältnis von 1800:1.

*Beispiel 7:*

11,15 g α-Acetamidozimtsäure in 125 ml Lösungsmittel (Äthanol/Benzol = 2:1) wurden in einen 250-ml-Kolben gegeben und mit 0,04 mmol des im Beispiel 5 hergestellten Katalysators versetzt. Unter Rühren wurde bei Raumtemperatur Wasserstoff durch die Lösung geleitet. Nach 4 h waren 64% des Substrats hydriert. Der Drehwert des getrockneten Produkts betrug

$$[\alpha]_D^{20} = +24° \ (c = 1/95\% \ EtOH)$$

*Beispiel 8:*

50 g α-Acetamidozimtsäure wurden in 250 ml einer 2:1-Mischung Äthanol/Benzol suspendiert, mit 31,9 mg des nach Beispiel 5 hergestellten Katalysators versetzt, und bei 15 bar in einem 500-ml-Autoklaven hydriert. Nach Aufnahme der theoretischen Wasserstoffmenge wurde, wie im Beispiel 6 beschrieben, aufgearbeitet. Es wurden 49,8 g Hydrierprodukt mit einem Drehwert von

$$[\alpha]_D^{20} = +43,5° \ (c = 1/95\% \ EtOH),$$

entsprechend einer optischen Ausbeute von 92% bei einem Substrat/Katalysator-Verhältnis von 6000:1, isoliert.

*Beispiel 9:*

3,0 g α-Acetamidoacrylsäure wurden in 45 ml einer Mischung aus 2 Volumenteilen Äthanol und 1 Volumenteil Benzol gelöst und mit 12,3 mg des im Beispiel 5 hergestellten Katalysators versetzt. Das Reaktionsgemisch wurde in einen evakuierten 100-ml-Autoklaven eingesaugt. Es wurden 14,8 bar $H_2$-Druck aufgepresst. Nach 2,5 h war die theoretische Wasserstoffmenge aufgenommen. Das, wie im Beispiel 6 beschrieben, aufgearbeitet N-Acetyl-(S)-alanin, 2,95 g, hatte einen Drehwert

$$[\alpha]_D^{20} = -56,0° \ (c = 2/H_2O),$$

entsprechend einer optischen Ausbeute von 84%, bezogen auf den aus der Literatur bekannten Drehwert

$$[\alpha]_D^{20} = -66,7° \ (c = 2/H_2O),$$

bei einem Substrat/Katalysator-Verhältnis von 1550:1.

*Beispiel 10:*

3,7 g p-Acetoxy-α-acetamidozimtsäure wurden in 45 ml absoluten Äthanol gelöst. Zu der Lösung wurden 6,75 mg des im Beispiel 5 hergestellten Katalysators gegeben und die Reaktionsmischung in einen evakuierten 100-ml-Autoklaven eingesaugt. Es wurden 21 bar $H_2$-Druck aufgepresst. Nach Aufnahme der theoretischen Wasserstoffmenge wurde wie üblich aufgearbeitet. Das isolierte p-Acetoxy-(S)-N-acetylphenylalanin hatte einen Drehwert

$$[\alpha]_D^{27} = +49,4° \ (c = 1/CH_3OH),$$

entsprechend einer optischen Ausbeute von 96%, bezogen auf den aus der Literatur bekannten Drehwert

$$[\alpha]_D^{27} = +51,5° \ (c = 1/CH_3OH)$$

*Beispiel 11:*

4,4 g p-Acetoxy-m-methoxy-α-acetamidozimtsäure wurden in 45 ml einer Mischung aus 2 Volumenteilen Äthanol und 1 Volumenteil Benzol gelöst und mit 12,9 mg des im Beispiel 5 hergestellten Katalysators versetzt. Das Reaktionsgemisch wurde in einen 100-ml-Autoklaven eingesaugt. Es wurden 14,9 bar $H_2$-Druck aufgepresst. Nach 2,5 h war die theoretische $H_2$-Menge aufgenommen. Das wie üblich aufgearbeitete Produkt, 4,3 g p-Acetoxy-m-methoxy-(S)-N-acetylphenylalanin hatte einen Drehwert

$$[\alpha]_D^{27} = +36,8° \ (c = 1/CH_3OH),$$

entsprechend einer optischen Ausbeute von 90,4%, bezogen auf den aus der Literatur bekannten Drehwert

$$[\alpha]_D^{27} = +40,7° \ (c = 1/CH_3OH)$$

Im NMR-Spektrum war kein Ausgangsmaterial mehr nachweisbar.

*Beispiel 12:*

*Darstellung von Cyclooctadien-[(S)-1,2-bis-(diphenylphosphino)-3-phenylpropan]rhodiumtetrafluoroborat*

Das Beispiel 5 wird wiederholt mit dem Unterschied, dass anstelle des (R)-1,2-Bis(diphenylphosphino)-3-phenylpropans das S-Isomere eingesetzt wird. Das Cyclooctadien-[(S)-1,2-bis(diphenylphosphino)-3-phenylpropan]rhodium-

tetrafluoroborat wird mit einer Ausbeute von 68% der Theorie erhalten.

*Elementaranalyse:*

| | | | |
|---|---|---|---|
| gefunden: | B 1,30 | C 62,42 | H 5,63% |
| berechnet: | B 1,37 | C 62,62 | H 5,38% |
| gefunden: | F 9,75 | Rh 13,17 | P 8,08% |
| berechnet: | F 9,66 | Rh 13,08 | P 7,88% |

*Beispiel 13:*

Zu 50 ml einer gesättigten Lösung von α-Acetamidozimtsäure in einer Mischung aus 2 Volumenteilen Äthanol und 1 Volumenteil Benzol wurden 7,9 mg des im Beispiel 12 hergestellten Katalysators gegeben. Die Reaktionsmischung wurde in einen evakuierten 100-ml-Autoklaven eingesaugt und mit 17 bar $H_2$-Druck beaufschlagt. Nach Aufarbeitung, wie im Beispiel 6 beschrieben, konnten 4,4 g N-Acetyl-(R)-phenylalanin mit einem Drehwert

$$[\alpha]_D^{20} = -46{,}5° \ (c = 1/95\% \ EtOH),$$

entsprechend einer optischen Ausbeute von 99%, isoliert werden.

*Beispiel 14:*

3,6 g p-Acetoxy-α-acetamidozimtsäure wurden in 45 ml absolutem Äthanol gelöst. Zu dieser Lösung wurden 7,1 mg des im Beispiel 12 hergestellten Katalysators gegeben. Nach Einsaugen der Reaktionslösung in einen evakuierten 100-ml-Autoklaven wurden 20 bar $H_2$-Druck aufgepresst. Nach Aufnahme der theoretischen Wasserstoffmenge wurde wie üblich aufgearbeitet. Es wurden 3,5 g p-Acetoxy-N-acetyl-(R)-phenylalanin mit einem Drehwert

$$[\alpha]_D^{27} = -48{,}4° \ (c = 1/CH_3OH),$$

entsprechend einer optischen Ausbeute von 94%, isoliert.

*Beispiel 15:*

*Darstellung* in situ *des Katalysators Cyclooctadien-[(R)-1,2-bis(diphenylphosphino)-3-methylbutan]rhodiumacetylacetonat*

Zur Herstellung der Katalysatorlösung wurden 0,1814 g des nach Beispiel 3 hergestellten (R)-1,2-Bis(diphenylphosphino)-3-methylbutans und 0,1272 g Cyclooctadienrhodiumacetylacetonat in 10 ml absolutem Äthanol bei Raumtemperatur gelöst.

*Beispiel 16:*

3,4 g α-Acetamidoacrylsäure wurden mit 0,5 ml der Katalysatorlösung von Beispiel 15 in 75 ml Äthanol in einem 250-ml-Kolben gelöst. Über einen Zeitraum von 6,5 h wurde Wasserstoffgas in die bei Raumtemperatur kräftig gerührte Lösung geleitet. Es wurde, wie im Beispiel 6 beschrieben, aufgearbeitet. Der Rückstand, 3,3 g, hatte einen Drehwert

$$[\alpha]_D^{20} = -59{,}1° \ (c = 2/H_2O),$$

was einer optischen Ausbeute von 88,6% entspricht.

Laut NMR-Spektrum war kein Ausgangsmaterial mehr vorhanden.

*Beispiel 17:*

3,0 g α-Benzamidozimtsäure wurden in 50 ml absolutem Äthanol gelöst. Es wurden 0,4 ml der Katalysatorlösung von Beispiel 15 hinzugegeben und die Reaktionsmischung in einen evakuierten und mehrmals mit Argon gespülten 100-ml-Autoklaven eingesaugt. Es wurden 20 bar $H_2$-Druck aufgepresst. Nach 2 h war die theoretische Menge an Wasserstoff aufgenommen. Das vom Katalysator und Lösungsmittel befreite N-Benzoyl-(S)-phenylalanin zeigte einen Drehwert

$$[\alpha]_D^{27} = -38{,}3° \ (c = 1/CH_3OH),$$

entsprechend einer optischen Ausbeute von 95%, bezogen auf den aus der Literatur bekannten Drehwert

$$[\alpha]_D^{27} = -40{,}3° \ (c = 1/CH_3OH)$$

Das NMR-Spektrum zeigte kein Ausgangsmaterial mehr.

*Beispiel 18:*

3,25 g p-Acetoxy-m-methoxy-α-acetamidozimtsäure wurden in 40 ml absolutem Äthanol gelöst und mit 0,5 ml der Katalysatorlösung von Beispiel 15 versetzt. Das Reaktionsgemisch wurde in einen 100-ml-Autoklaven eingesaugt. Es wurden 6,6 bar $H_2$-Druck aufgepresst. Nach 6,25 h war die Reaktion beendet. Das wie üblich aufgearbeitete p-Acetoxy-m-methoxy-N-acetyl-(S)-phenylalanin, 3,2 g, hatte einen Drehwert

$$[\alpha]_D^{27} = +35{,}8°,$$

entsprechend einer optischen Ausbeute von 88%, wenn man den Drehwert des reinen Enantiomeren mit 40,7° (c = 1/$CH_3OH$) zugrunde legt.

Das NMR-Spektrum zeigte kein Ausgangsmaterial mehr.

*Beispiel 19:*

*Darstellung* in situ *des Katalysators Cyclooctadien[(S)-1,2-bis(diphenylphosphino)-3-methylbutan]rhodiumacetylacetonat*

Zur Herstellung der Katalysatorlösung wurden 0,2083 g des nach Beispiel 4 hergestellten (S)-1,2-Bis(diphenylphosphino)-3-methylbutans und 0,2970 g Cyclooctadienrhodiumacetylacetonat in 10 ml absolutem Äthanol bei Raumtemperatur gelöst.

*Beispiel 20:*

3,5 g α-Acetamidoacrylsäure wurden mit 0,2 ml der im Beispiel 19 hergestellten Katalysatorlösung in einem 250-ml-Kolben in 75 ml absolutem Äthanol gelöst. Über einen Zeitraum von 6,5 h wurde Wasserstoffgas in die bei Raumtemperatur kräftig gerührte Lösung geleitet. Es wurde, wie im Beispiel 6 beschrieben, aufgearbeitet. Das wie üblich aufgearbeitete N-Acetyl-(R)-alanin, 3,4 g, hatte einen Drehwert

$$[\alpha]_D^{20} = +59{,}0° \ (c = 2/H_2O),$$

entsprechend einer optischen Ausbeute von 88,5%.

*Beispiel 21:*

3,2 g α-Benzamidozimtsäure wurden in 40 ml absolutem Äthanol gelöst und mit 0,3 ml der im Beispiel 19 hergestellten Katalysatorlösung versetzt. Das Reaktionsgemisch wurde in einen 100-ml-Autoklaven eingesaugt. Es wurden 6 bar $H_2$-Druck aufgepresst. Nach Aufnahme der theoretischen Wasserstoffmenge wurde wie üblich aufgearbeitet. Es wurden 3,15 g N-Benzoyl-(R)-phenylalanin mit dem Drehwert

$$[\alpha]_D^{27} = +38{,}6° \ (c = 1/CH_3OH),$$

entsprechend einer optischen Ausbeute von 95,8% isoliert.

In NMR-Spektrum war kein Ausgangsmaterial mehr zu erkennen.

## Patentansprüche

1. Optisch aktive 1,2-Bis(diphenylphosphino)-verbindungen der allgemeinen Formel

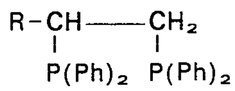

(I)

in der Ph einen Phenylrest, und R einen Isopropyl-, einen Isobutyl-, einen sekundären Butyl- oder einen Benzylrest bedeuten.

2. Metallkomplexe des Rhodiums, die *in situ* hergestellt worden sind durch Umsetzung einer Verbindung gemäss Anspruch 1

mit einem Komplex der allgemeinen Formel

$$[Rh(en)_2X]_2 \quad (II)$$

in der $(en)_2$ zwei Moleküle eines Monoolefins oder ein Molekül eines Diolefins, und X Chlor, Brom oder Jod bedeuten, im molaren Verhältnis 2:1, oder

mit einem Komplex der allgemeinen Formel

$$[Rh(en)_2Y] \quad (III)$$

in der $(en)_2$ die angegebene Bedeutung hat, und Y einen Acetylacetonat- oder Carboxylatrest bedeutet, im molaren Verhältnis 1:1.

3. Metallkomplexe des Rhodiums mit der allgemeinen Formel

$$[Rh(en)_2A]^+ Z^- \quad (IV)$$

in der $(en)_2$ zwei Moleküle eines Monoolefins oder ein Molekül eines Diolefins, A eine Verbindung gemäss Anspruch 1, und $Z^-$ ein Tetrafluoroborat-, Hexafluorophosphat- oder Perchloratanion bedeuten.

4. Verwendung von Metallkomplexen gemäss einem der Ansprüche 2 oder 3 als Katalysator für die homogene asymmetrische Hydrierung unsubstituierter oder β-substituierter α-Acylamidoacrylsäure bzw. deren Derivate.

## Revendications

1. Composés 1,2-bis-diphénylphosphiniques optiquement actifs de formule générale:

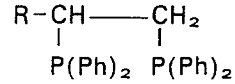

(I)

où Ph signifie un groupe phényle, et R un groupe isopropyle, isobutyle, sec.-butyle ou benzyle.

2. Complexes métalliques du rhodium, qui ont été obtenus *in situ* par réaction d'un composé selon la revendication 1, avec un complexe de formule générale:

$$[Rh(en)_2X]_2 \quad (II)$$

où $(en)_2$ représente deux molécules d'une monooléfine ou une molécule d'une dioléfine, et X un chlore, brome ou iode, dans un rapport molaire de 2:1, ou avec un complexe de formule générale:

$$[Rh(en)_2Y] \quad (III)$$

où $(en)_2$ a la même signification, et où Y représente un groupe acétylacétonate ou carboxylate, dans un rapport molaire de 1:1.

3. Complexes métalliques du rhodium de formule générale:

$$[Rh(en)_2A]^+ Z^- \quad (IV)$$

où $(en)_2$ représente deux molécules d'une monooléfine ou une molécule d'une dioléfine, A un composé selon la revendication 1, et $Z^-$ un anion tétrafluoroborate, hexafluorophosphate ou perchlorate.

4. Utilisation des complexes métalliques selon l'une des revendications 2 ou 3 comme catalyseurs pour l'hydrogénation homogène asymétrique d'acides α-acylamidoacryliques, non substitués ou β-substitués, ou de leurs dérivés.

## Claims

1. Optically active 1,2-bisdiphenylphosphino compounds corresponding to the general formula:

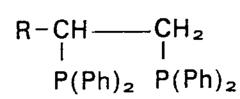

(I)

wherein Ph represents a phenyl radical, and R represents an isopropyl radical, an isobutyl radical, a secondary butyl radical or a benzyl radical.

2. Metal complexes of rhodium which are produced *in situ* by reacting a compound according to claim 1 with a complex corresponding to the general formula:

$$[Rh(en)_2X]_2 \quad (II)$$

wherein $(en)_2$ represents two molecules of a

monoolefin or one molecule of a diolefin, and X represents chlorine, bromine or iodine, in a molar ratio of 2:1, or with a complex corresponding to the general formula:

$$[Rh(en)_2Y] \qquad (III)$$

wherein $(en)_2$ is defined as above, and Y represents an acetylacetonate radical or a carboxylate radical, in a molar ratio of 1:1.

3. Metal complexes of rhodium having the general formula:

$$[Rh(en)_2A]^+ Z^- \qquad (IV)$$

wherein $(en)_2$ represents two molecules of a monoolefin or one molecule of a diolefin, A represents a compound according to claim 1, and $Z^-$ represents a tetrafluoroborate anion, a hexafluorophosphate anion or a perchlorate anion.

4. The use of metal complexes according to one of claims 2 or 3 as catalyst for the homogeneous asymmetric hydrogenation of unsubstituted or β-substituted α-acylamidoacrylic acid or derivatives thereof.